# EUROPEAN PATENT SPECIFICATION

(11) **EP 1 467 702 B1**
(45) Date of publication and mention of the grant of the patent: **23.05.2018**
(21) Application number: 02804959.1
(22) Date of filing: 13.12.2002
(51) Int. Cl.: A61Q 19/00, A61K 8/891, A61K 8/58, A61K 8/81, A61P 17/02

(54) **SCAR MANAGEMENT COMPOSITION**
ZUSAMMENSETZUNG ZUR NARBENBEHANDLUNG
COMPOSITION DE SOIN DE CICATRICES

(30) Priority: 14.12.2001 GB 0129886
(43) Date of publication of application: 20.10.2004
(73) Proprietor: Medtrade Products Ltd., Crewe CW1 6GL (GB)
(72) Inventor: HARDY, Craig, Julian, Cheshire CW3 0AY (GB)
(74) Representative: Wilson Gunn
(86) International application number: PCT/GB2002/005686
(87) International publication number: WO 2003/051326

(56) References cited:
- EP-A- 1 051 965
- WO-A-00/61076
- WO-A-01/22923
- WO-A-01/37782
- WO-A-95/05800
- WO-A-96/14054
- WO-A-98/07404
- US-A- 4 920 158
- US-A- 5 380 528
- US-A- 5 567 426
- US-A- 5 795 575

## Description

The present invention relates to a composition which finds particular utility in scar management.

It has been known for some years that the cosmetic severity of tissue scarring as a result of medical treatment and/or injury can be greatly reduced by the application of a silicone-containing sheet to the skin's surface. The mode of action remains unclear.

The first silicone-containing sheets used in order to reduce the visual severity of tissue scarring, based on a rubber containing formulation, were developed approximately 25 years ago and met with limited success since they were difficult to use. These silicone rubber sheets were secured on the skin surface with adhesive tape thereby forming a sealed enclosure. Typically, the silicone rubber sheet would be secured in place for 30 days during which time the skin would 'remodel' itself to a large degree giving rise to a decrease in the cosmetic severity of the scar tissue.

Several theories have been postulated regarding the mode of action of the silicone layers. These include the moisture barrier properties of silicone, the leaching of low molecular weight silicone into the skin and the effect of the ionic content of the silicone.

Despite these silicone rubber sheets being expensive, they were widely used even though the location of their use on the skin surface was limited by their lack of flexibility.

The aforesaid silicone rubber sheets have since been replaced with softer self-adhesive silicone gel sheets, which possess a greater degree of flexibility by virtue of their polymeric oil-containing network structure. Therefore, although easier to apply, their use remains limited as a result of their sheet-like nature.

More recently liquid silicone compositions have been introduced. These are supplied in a tube and are specifically designed to be applied to awkward areas of the skin's surface. Examples of said compositions include KELO-COTE (TM) or with a roller ball applicator, CICACARE (TM). Unlike the previously described sheets, which are intended to be worn for a period of approximately 30 days, these liquid silicone compositions require re-application every few hours as the silicone layer located over the wound is thinner. However, these compositions remain sticky and tacky after application, some fail to dry, whilst others take 10 to 30 minutes to dry.

US 5795575 relates to topical medicaments having cicatrizing activity and discloses a method for increasing the number of capillaries in damaged skin during healing of the skin.

It is desirable therefore to provide a composition which delivers the aforesaid cosmetic scar severity reduction and which is more convenient to use.

According to a first aspect of the present invention there is provided a composition as defined by Claim 1.

Advantageously, the composition of the present invention is in the form of a surprisingly stable gel which is easy to manufacture and as such is suitable for application to all skin areas, but it is particularly useful for the application to areas to which it is difficult to apply sheet material.

By gel it is meant a viscous, at least partially rigid material which can be manipulated to form a thin film.

A further advantage of the composition of the present invention lies with its ability to deliver moisture to the wound immediately on application by virtue of the composition's water content, thus, eliminating the need, as is the case with prior art silicone-containing sheets, for moisture to develop under the sheet thus giving an immediate skin softening and moisturising effect. The water content of the compositions of the present invention ultimately results in a more efficient and effective treatment.

A still further advantage of the composition of the present invention is its rapid drying time, typically of about 2 minutes, thereby eliminating the undesirable tack of previously used liquid compositions which have a much longer drying time, typically in the order of 10 to 30 minutes.

Surprisingly, the composition of the present invention results in a greater reduction in the visual severity of a scar than that seen with previous methods of scar management.

Accordingly, the composition of the present invention is not only much easier and quicker to use than its predecessors but results in a greater reduction in the visual severity of scar tissue.

The silicone component constitutes from 5% to 80%, most preferably from 5% to 30% by weight, of the composition of the present invention.

The silicone component for use in the composition of the present invention includes any of the following either alone or in combination:- silicone, silicone oil, silicone gels, silicone grease, silicone elastomer, or cross polymer, silicone gum, silicone adhesive, silicone emulsifier, silicone wax emulsifier and silicone copolymer.

Preferably, the silicone component of the present invention is a silicone elastomer and/or a silicone gum. Advantageously this gives rise to a composition having a longer wear time than currently available scar management products. Table 3, shows how the inclusion of silicone elastomer increases the wear time of the product.

The thickening agent(s) preferably constitutes from 0.01% to 50%, more preferably from 0.01% to 10%, still more preferably from 0.01% to 0.99% and most preferably from 0.01% to 0.75% by weight, of the composition of the present invention.

Suitable thickening agents include any of the following either alone or in combination:- polyacrylic acid thickeners such as Carbopol, polyacrylic acid derivatives, polyvinyl alcohol (PVA, PVOH), polyvinyl pyrrolidine (PVP), PVP/PVA copolymer, hydroxyethyl cellulose, acrylatepolymer, alkyl acrylates cross copolymer, hydrolysed protein, cetearyl alcohol, plant polysaccharide, microbial polysaccharide, animal polysaccharide, carboxymethyl cellulose, methyl cellulose, alginate, chitin/chitosan, hyaluronic acid, collagen, pectin, gelatine, starch and starch copolymer.

Preferably water constitutes from 2% to 99.8%, by weight of the composition of the present invention.

The composition of the present invention contains at least one volatile organic solvent being a volatile silicone solvent.

The said solvent may constitute from 5% to 80% and most preferably from 10.1% to 80% by weight, of the composition of the present invention.

Preferably the composition of the present invention shall comprise at least one neutralising agent which can be mineral and/or organic in nature. Any suitable base may be used, for example sodium hydroxide, potassium hydroxide, triethanolamine and any suitable inorganic base.

In addition to the foregoing the composition of the present invention may also comprise any of the following, either alone or in combination:- active agents such as antibacterial agents, surfactants, antimicrobial agents, antimicrobial essential oil, skin occlusion agents, copolymers and skin moisturisers, skin healing agents, scar healing agents, growth factors, or any compound which effects the level of growth factors in the skin, wound or scar, herbal agents, plant oils, essential oils or other plant extracts.

There is also described a method of preparing a scar management composition comprising mixing together at least two formulations wherein the first formulation comprises water and at least one thickening agent and the second formulation comprises at least one silicone component.

The first formulation may be a viscous water-based hydrogel which is formed when water containing at least one thickening agent typically in total 1%, by weight, is vigorously stirred. The resulting viscous hydrogel is subsequently neutralised using sodium hydroxide, for example. The first formulation may optionally contain at least one silicone component. The first formulation may also contain active agents such as antibacterial agents, antimicrobial agents and/or skin moisturising agents.

The second formulation may be a mixture of two independent parts.

The first part of the second formulation may comprise volatile organic solvent(s) and any organic solvent soluble components such as one or more skin moisturisers.

The second part of the second formulation may comprise any of the aforesaid silicone components along with any surfactant and copolymer.

During preparation of the composition of the present invention the first formulation may be added to the second formulation with vigorous mixing, again until a homogeneous hydrogel ensues.

In order that the present invention be more readily understood the composition of the present invention will now be described further by way of example only with relation to the following examples:-

### Example 1

Silmogen Masterbatch, ethanol and nusil silicone grease were pre-mixed to form the second formulation as a homogeneous mixture.

Carbopol ultrex was added to water with continuous stirring, furnishing the first formulation as a homogeneous hydrogel which was neutralised with 2% aqueous solution of sodium hydroxide.

The first formulation was then added to the second formulation with vigorous stirring to furnish a homogenous gel, the composition of the present invention.

| Formulation | Component | Function | Quantity |
|---|---|---|---|
| First | Water | Solvent | 200g |
| | Carbopol Ultrex | Thickening Agent | 2g |
| | NaOH (2% solution) | Neutralising Agent | 2 wt% of total comp. A |
| Second | Silmogen Masterbatch | Silicone Component | 54g |
| | Ethanol | Organic Solvent | 18g |
| | Nusil | Silicone Grease | 6g |

NB: Drying time (average of 3 tests) 93 seconds.

### Example 2

The composition was prepared in the aforesaid manner

| Formulation | Component | Function | Quantity |
|---|---|---|---|
| First | Water | Solvent | 200g |
| | Carbopol 914 | Thickening Agent | 1.5g |
| | Neutrol TE | Neutralising Agent | to pH 7 |
| Second | Ethanol | Organic Solvent | 100g |
| | Silmogen Carrier | Silicone Solvent | 100g |
| | Dimethicone Blend | Silicone Component | 20-25g |

| | | | |
|---|---|---|---|
| NB: Drying time 23 seconds. | | | |

### Example 3

The composition was prepared in the aforesaid manner

| Formulation | Component | Function | Quantity |
|---|---|---|---|
| First | Water | Solvent | 190g |
| | Medical Grade Aloe Vera Gel | Moisturising Agent | 10g |
| | Carbopol 914 | Thickening Agent | 5g |
| | Neutrol TE | Neutralising Agent | to pH 7 |
| Second | Ethanol | Organic Solvent | 200g |
| | Silmogen Carrier | Silicone Solvent | 125g |
| | Dimethicone Blend | Silicone Component | 20-75g |

| | | | |
|---|---|---|---|
| NB: Drying time 98 seconds. | | | |

### Example 4

The composition was prepared in the aforesaid manner

| Formulation | Component | Function | Quantity |
|---|---|---|---|
| First | Water | Solvent | 200g |
| | Carbol 914 | Thickening Agent | 5g |
| | Salicylic Acid | Antibacterial Agent | 0.5g |
| | Neutrol TE | Neutralising Agent | to pH 7 |
| Second | Ethanol | Organic Solvent | 200g |
| | Silmogen Carrier | Silicone Solvent | 125g |
| | Dimethicone Blend | Silicone Component | 20-75g |

NB: Drying time - not available.

### Example 5

The composition was prepared in the aforesaid manner

| Formulation | Component | Function | Quantity (g) |
|---|---|---|---|
| First | Water | Solvent | 247.55 |
| | Ethanol | Organic Solvent | 108.35 |
| | Carbopol 980NF | Thickener | 3.20 |
| | Nipagin M | Preservative | 0.29 |
| | Nipasol M | Preservative | 0.07 |
| Second | Elastomer 10 | Silicone component | 33.85 |
| | Cyclomethicone 5 | Silicone component | 22.44 |
| | Isopropyl Myristate | Solubilising agent | 4.25 |

The composition was neutralised to between pH 7.3 and 7.6 using sodium hydroxide.
N.B. Drying time 80 seconds.

### Example 6 (Reference Example)

The composition was prepared in the aforesaid manner

| Formulation | Component | Function | Quantity (g) |
|---|---|---|---|
| First | Water | Solvent | 247.55 |
| | Ethanol | Organic Solvent | 108.35 |
| | Carbopol 980NF | Thickening Agent | 4.6 |
| | Nipagin M | Preservative | 0.29 |
| | Niasol M | Preservative | 0.07 |
| Second | Dow Corning silicone fluid | Silicone component | 56.5 |

Drying time - not available.

### Example 7 (Reference Example)

Unlike the previous examples the composition described below does not contain any organic solvents.

Carbopol 940 was added to water, with vigorous stirring. The resulting hydrogel was neutralised using sodium hydroxide, thus furnishing the first formulation.

The first formulation was then stirred vigorously while dimethicone blend 20, the second formulation, was introduced.

The resulting gel was mixed for three minutes.

| Formulation | Component | Function | Quantity |
|---|---|---|---|
| First | Water | Solvent | 200g |
| | Carbopol 940 | Thickening Agent | 1.5g |
| | NaOH | Neutralising Agent | to pH 7 |
| Second | Dimethicone blend 20 | Silicone Component | 20g |

NB: Drying time 435 seconds.

### Example 8 (Reference example)

In addition to the components of examples 1 to 4 the composition described below contains emulsifiers.

Carbopol 980 was added to water. This mixture was then pre-heated to 50°C and a mixture of stearyl alcohol and trimethylstearyloxysilane was introduced providing the first formulation as an emulsion, which was then allowed to cool.

Dimethicone blend 20 and ethanol were mixed together resulting in the second formulation.

The second formulation was then introduced to a vigorously stirred first formulation.

| Formulation | Component | Function | Quantity |
|---|---|---|---|
| First | Water | Solvent | 168g |
| | Carbopoly 980 | Thickening Agent | 2g |
| | Silky wax 10 (a mixture of stearyl alcohol and trimethylstearyloxy silane) | Silicone component | 2g |
| Second | Ethanol | Organic Solvent | 10% of component phase A |
| | Dimethicone blend 20 | Silicone Component | 10% of component phase A |

NB: Drying time 360 seconds.

Clinical case studies have been performed to assess the effectiveness of the scar management compositions of the present invention.

In the following case study the patient was asked using the guidelines exemplified in Table 1, to comment as to the appearance and feel of each scar following treatment with compositions described herein.

**Table 1.**

| **Questions** | **Results** |
|---|---|
| 1. Has the scar improved since the last assessment? | Yes or No |
| 2. Rate its appearance on a scale of 1 to 5 (with 1 as the best and 5 as the worst)? | 1 to 5 |
| 3. Rate its flexibility on a scale of 1 to 4 (with 1 as the best and 5 as the worst)? | 1 to 5 |
| 4. Rate its softness on a scale of 1 to 5 (with 1 as the best and 5 as the worst)? | 1 to 5 |
| 5. Does it feel irritated? Rate this on a scale of 1 to 5 (with 1 as the best and 5 as the worst)? | 1 to 5 |
| 6. Is it better or worse than the scar it was originally paired against? | Better or Worse |

### Case Study

A patient had four fatty cysts surgically removed from under the surface layer of the skin on the arm. Following surgery the wounds were sutured closed and dressings with Jelonet ™ and a gauze bandage were applied thereto, The patient was asked to minimise the movement of the arm and keep it elevated for 24hours after the operation.

The sutures were removed 10 days after surgery.

Three days after the removal of the sutures the scars appeared dry and red.

The patient was asked to apply a scar management composition according to the present invention to two of the scars (scar 1 and scar 3) each morning and after every bath or shower.

The other two scars (scar 2 and scar 4) were not treated.

The scars were assessed 12 days, 26 days and 68 days from the first treatment.

The results of the clinical case study are shown in Table 2.

**Table 2**

| | After 12 days | | | | After 26 days | | | | After 68 days | | | |
|---|---|---|---|---|---|---|---|---|---|---|---|---|
| Question | scar 1 | scar 2 | scar 3 | scar 4 | scar 1 | scar 2 | scar 3 | scar 4 | scar 1 | scar 2 | scar 3 | scar 4 |
| 1.. | Yes | Yes | Yes | Yes | Yes | No, maybe slightly softer | Yes | No, it got worse, more red and more raised | Yes | No | Yes | No |
| 2. | 3 | 3 | 4 | 5 | 2 | 3 | 3 | 5 | 1 | 3 | 2 | 5 |
| 3. | 2 | 4 | 3 | 3 | 1 | 3 | 2 | 2 | 1 | 2 | 1 | 3 |
| 4. | 2 | 4 | 3 | 3 | 1 | 3 | 2 | 2 | 1 | 2 | 1 | 3 |
| 5. | 3 | 3 | 2 | 4 | 1 | 2 | 1 | 5 | 1 | 2 | 1 | 4 |
| 6. | Very slightly better than scar 2 | Very slightly worse than scar 1 | Same as scar 4 | Same as scar 3 | Better in colour and softness/ flexibility than scar 2 | Worse in colour and softness/ flexibility than scar 1 | Considerably betterthan scar 4 in colour | Considerably worse than scar 3 in colour, height and irritation | Considerably better in colour and softness/ flexibility than scar 2 | Worse in colour and softness/ flexibility than scar 1 | Considerably better than scar 4 in colour, height and irritation | Considerably worse than scar 3 in colour, height and irritation |

**Table 3**

| Example | Format | Elastomer or gum present? | Wear Time (hours) | | | | | | |
|---|---|---|---|---|---|---|---|---|---|
| | | | 1 | 2 | 4 | 6 | 8 | 10 | 12 |
| Competitor Product Kelocote™ (control) | - | Neither | Pas s | Pass | Fail | | | | |
| Example 6 | Hydrogel | Neither | Pas s | Pass | Fail | | | | |
| Example 5 | Hydrogel | Elastomer | Pas s | Pass | Pass | Pass | Pass | Pass | Fail |

### Test method summary

0.07g of test material is applied to a 5cm² area of skin.

The area is wetted with water after 1 hour, 2 hours, 4 hours and then every 2 hours.

The product 'passes' if the water after forms an elevated bubble indicated that a significant amount of silicone is still present.

The product 'fails' if the water does not form a elevated bubble indicated that little if any silicone is still present.

It is of course to be understood that the invention is not intended to be restricted to the details of the above embodiments which are described by way of example only.

## Claims

1. A composition in the form of a hydrogel comprising a silicone component which includes any of the following either alone or in combination: silicone, silicone oil, silicone gels, silicone grease, silicone elastomer, silicone cross-polymer, silicone gum, silicone adhesive, silicone emulsifier, silicone wax emulsifier and silicone co-polymer, wherein the silicone component constitutes from 5% to 80% by weight of the total composition; at least one volatile organic solvent being a volatile silicone solvent; water, and at least one thickening agent, for use in the treatment of scar tissue.

2. A composition according to any preceding claim, wherein said at least one thickening agent constitutes from 0.01% to 50%, by weight, of the total composition.

3. A composition according to claim 2, wherein said at least one thickening agent constitutes from 0.01% to 10%, by weight, of the total composition.

4. A composition according to claim 3, wherein said at least one thickening agent constitutes from 0.01% to 0.99%, by weight, of the total composition.

5. A composition according to claim 4 wherein said at least one thickening agent constitutes from 0.01% to 0.75%, by weight, of the total composition.

6. A composition according to any preceding claim, wherein said at least one thickening agent comprises any of the following either alone or in combination:-polyacrylic acid thickener, polyacrylic acid derivative, polyvinyl alcohol, polyvinyl pyrrolidine, polyvinyl pyrrolidine/polyvinyl acrylate copolymer, hydroxyethyl cellulose, acrylatepolymer, alkyl acrylate cross copolymer, hydrolysed protein, cetearyl alcohol, plant polysaccharide, microbial polysaccharide, animal polysaccharide, carboxymethyl cellulose, methyl cellulose, alginate, chitin/chitosan, hyaluronic acid, collagen, pectin, gelatine, starch or starch copolymer.

7. A composition according to any preceding claim, wherein the water constitutes from 2% to 99.8%, by weight, of the total composition.

8. A composition according to any preceding claim, wherein said at least one volatile organic solvent constitutes from 5% to 80%, by weight, of the total composition.

9. A composition according to claim 8, wherein said at least one volatile organic solvent constitutes from 10.1% to 80%, by weight, of the total composition.

10. A composition according to any preceding claim, wherein the composition comprises at least one neutralising agent.

11. A composition according to claim 10, wherein the said at least one neutralising agent is mineral and/or organic in nature.

12. A composition according to claim 10, wherein the said at least one neutralising agent comprises any of the following either alone or in combination:- sodium hydroxide, potassium hydroxide or triethanolamine.

13. A composition according to any preceding claim, wherein the composition further comprises any of the following either alone or in combination; antibacterial agent, antimicrobial agent, antimicrobial essential oil, skin occlusion agents, skin moisturizers, skin healing agents or scar healing agents, growth factor or any compound which effects the level of growth factors in the skin, wound or scar, herbal agents, plant oils, essential oils or other plant extract.

14. A composition according to any preceding claim, wherein the composition further comprises skin occlusion agents.

15. A composition according to any preceding claim, wherein the composition further comprises at least one skin moisturizer.

16. A composition according to any preceding claim, wherein the composition further comprises any of the following either alone or in combination:- skin healing agents, scar healing agents, growth factors or any compound which affects the level of growth factors in the skin, wound or scar.

17. A composition according to any preceding claim, wherein the composition further comprises any of the following either alone or in combination:- herbal agents, plant oils, essential oils or other plant extracts.

## Patentansprüche

1. Zusammensetzung in Form eines Hydrogels umfassend eine Silikonkomponente, die eines der folgenden entweder allein oder in Kombination umfasst: Silikon, Silikonöl, Silikongele, Silikonfett, Silikonelastomer, Silikon-Kreuzpolymer, Silikongummi, Silikonklebstoff, Silikonemulgator, Silikonwachsemulgator und Silikoncopolymer, wobei die Silikonkomponente von 5 Gew.-% bis 80 Gew.-% der Gesamtzusammensetzung bildet; zumindest ein flüchtiges organisches Lösungsmittel, das ein flüchtiges Silikonlösungsmittel ist; Wasser, und zumindest ein Verdickungsmittel, zur Verwendung bei der Behandlung von Narbengewebe.

2. Zusammensetzung nach einem der vorhergehenden Ansprüche, wobei das zumindest eine Verdickungsmittel von 0,01 Gew.-% bis 50 Gew.-% der Gesamtzusammensetzung bildet.

3. Zusammensetzung nach Anspruch 2, wobei das zumindest eine Verdickungsmittel von 0,01 Gew.-% bis 10 Gew.-% der Gesamtzusammensetzung bildet.

4. Zusammensetzung nach Anspruch 3, wobei das zumindest eine Verdickungsmittel von 0,01 Gew.-% bis 0,99 Gew.-% der Gesamtzusammensetzung bildet.

5. Zusammensetzung nach Anspruch 4, wobei das zumindest eine Verdickungsmittel von 0,01 Gew.-% bis 0,75 Gew.-% der Gesamtzusammensetzung bildet.

6. Zusammensetzung nach einem der vorhergehenden Ansprüche, wobei das zumindest eine Verdickungsmittel eines der folgenden entweder allein oder in Kombination umfasst: - Polyacrylsäureverdicker, Polyacrylsäurederivat, Polyvinylalkohol, Po lyvinylpyrro lidin, Polyvinylpyrrolidin/Polyvinylacrylat-Copolymer, Hydroxyethylzellulose, Acrylatpolymer, Alkylacrylat-Kreuzcopolymer, hydrolysiertes Protein, Cetearylalkohol, pflanzliches Polysaccharid, mikrobielles Polysaccharid, tierisches Polysaccharid, Carboxymethylzellulose, Methylzellulose, Alginat, Chitin/Chitosan, Hyaluronsäure, Kollagen, Pektin, Gelatine, Stärke oder Stärke-Copolymer.

7. Zusammensetzung nach einem der vorhergehenden Ansprüche, wobei das Wasser von 2 Gew.-% bis 99,8 Gew.-% der Gesamtzusammensetzung bildet.

8. Zusammensetzung nach einem der vorhergehenden Ansprüche, wobei das zumindest eine flüchtige organische Lösungsmittel von 5 Gew.-% bis 80 Gew.-% der Gesamtzusammensetzung bildet.

9. Zusammensetzung nach Anspruch 8, wobei das zumindest eine flüchtige organische Lösungsmittel von 10,1 Gew.-% bis 80 Gew.-% der Gesamtzusammensetzung bildet.

10. Zusammensetzung nach einem der vorhergehenden Ansprüche, wobei die Zusammensetzung zumindest ein Neutralisationsmittel umfasst.

11. Zusammensetzung nach Anspruch 10, wobei das zumindest eine Neutralisationsmittel mineralischer und/oder organischer Natur ist.

12. Zusammensetzung nach Anspruch 10, wobei das zumindest eine Neutralisationsmittel eines der folgenden entweder allein oder in Kombination umfasst: - Natriumhydroxid, Kaliumhydroxid oder Triethanolamin.

13. Zusammensetzung nach einem der vorhergehenden Ansprüche, wobei die Zusammensetzung ferner eines der folgenden entweder allein oder in Kombination umfasst; einen antibakteriellen Wirkstoff, einen antimikrobiellen Wirkstoff, antimikrobielles ätherisches Öl, hautverschließende Wirkstoffe, Hautbefeuchtungsmittel, hautheilende Wirkstoffe oder narbenheilende Wirkstoffe, Wachstumsfaktor oder eine Verbindung, die den Spiegel von Wachstumsfaktoren in der Haut, Wunde oder Narbe beeinflusst, pflanzliche Wirkstoffe, Pflanzenöle, ätherische Öle oder andere Pflanzenextrakte.

14. Zusammensetzung nach einem der vorhergehenden Ansprüche, wobei die Zusammensetzung ferner hautverschließende Wirkstoffe umfasst.

15. Zusammensetzung nach einem der vorhergehenden Ansprüche, wobei die Zusammensetzung ferner zumindest ein Hautbefeuchtungsmittel umfasst.

16. Zusammensetzung nach einem der vorhergehenden Ansprüche, wobei die Zusammensetzung ferner eines der folgenden entweder allein oder in Kombination umfasst: - hautheilende Wirkstoffe, narbenheilende Wirkstoffe, Wachstumsfaktoren oder eine Verbindung, die den Spiegel von Wachstumsfaktoren in der Haut, Wunde oder Narbe beeinflusst.

17. Zusammensetzung nach einem der vorhergehenden Ansprüche, wobei die Zusammensetzung ferner eines der folgenden entweder allein oder in Kombination umfasst: - pflanzliche Wirkstoffe, Pflanzenöle, ätherische Öle oder andere Pflanzenextrakte.

## Revendications

1. Composition sous la forme d'un hydrogel, comprenant un constituant de silicone, qui inclut l'un quelconque des suivants seul ou en combinaison : silicone, huile de silicone, gels de silicone, graisse de silicone, élastomère de silicone, polymère réticulé de silicone, gomme de silicone, adhésif de silicone, émulsionnant de silicone, émulsionnant de cire de silicone et copolymère de silicone, dans laquelle le constituant de silicone représente de 5% à 80% du poids de la composition totale, au moins un solvant organique volatil étant un solvant de silicone volatil ; eau et au moins un agent épaississant, à utiliser dans le traitement d'un tissu ayant une cicatrice.

2. Composition suivant l'une quelconque des revendications précédentes, dans laquelle le au moins un agent épaississant représente de 0,01% à 50%, en poids, de la composition totale.

3. Composition suivant la revendication 2, dans laquelle un agent épaississant représente de 0,01% à 10%, en poids, de la composition totale.

4. Composition suivant la revendication 3, dans laquelle le au moins un agent épaississant représente de 0,01% à 0,99% en poids de la composition totale.

5. Composition suivant la revendication 4, dans laquelle le au moins un agent épaississant représente de 0.01% à 0,75% en poids de la composition totale.

6. Composition suivant l'une quelconque des revendications précédentes, dans laquelle le au moins un agent épaississant comprend l'un quelconque des suivants seul ou en combinaison : épaississant à base de poly(acide acrylique), dérivé de poly(acide acrylique), alcool polyvinylique, polyvinyl pyrrolidine, copolymère de polyvinyl pyrrolidine/ poly(acrylate de vinyle), hydroxyéthylcellulose, polymère d'acrylate, copolymère réticulé d'acrylate d'alcoyle, protéine hydrolysée, alcool cétéarylique, polysaccharide végétal, polysaccharide microbien, polysaccharide animal, carboxyméthylcellulose, méthylcellulose, alginate, chitine/ chitosane, acide hyaluronique, collagène, pectine, gélatine, amidon ou copolymère d'amidon.

7. Composition suivant l'une quelconque des revendications précédentes, dans laquelle l'eau représente de 2% à 99,8% en poids de la composition totale.

8. Composition suivant l'une quelconque des revendications précédentes, dans laquelle le au moins un solvant organique volatil représente de 5% à 80%, en poids, de la composition totale.

9. Composition suivant la revendication 8, dans laquelle le au moins un solvant organique volatil représente de 10,1% à 80% en poids de la composition totale.

10. Composition suivant l'une quelconque des revendications précédentes, dans laquelle la composition comprend au moins un agent de neutralisation.

11. Composition suivant la revendication 10, dans laquelle le au moins un agent de neutralisation est de nature minérale et/ou organique.

12. Composition suivant la revendication 10, dans laquelle le au moins un agent de neutralisation comprend l'un quelconque des suivants seul ou en combinaison : hydroxyde de sodium, hydroxyde de potassium ou triéthanolamine.

13. Composition suivant l'une quelconque des revendications précédentes, dans laquelle la composition comprend, en outre, l'un quelconque des suivants seul ou en combinaison : agent antibactérien, agent antimicrobien, huile essentielle antimicrobienne, agents d'occlusion de la peau, agents d'humidification de la peau, agents de soin de la peau ou agents de cicatrisation, facteur de croissance ou tout composé qui affecte le taux de facteur de croissance dans la peau, une plaie ou une cicatrice, simples, huiles végétales, huiles essentielles ou d'autres extraits de plante.

14. Composition suivant l'une quelconque des revendications précédentes, dans laquelle la composition comprend, en outre, des agents d'occlusion de la peau.

15. Composition suivant l'une quelconque des revendications précédentes, dans laquelle la composition comprend, en outre, au moins un agent d'humidification de la peau.

16. Composition suivant l'une quelconque des revendications précédentes, dans laquelle la composition comprend, en outre, l'un quelconque des suivants seul ou en combinaison : des agents de soin de la peau, des agents cicatrisants, des facteurs de croissance ou tout composé qui affecte le taux de croissance dans la peau, une plaie ou une cicatrice.

17. Composition suivant l'une quelconque des revendications précédentes, dans laquelle la composition comprend, en outre, l'un quelconque des suivants seul ou en combinaison : des huiles végétales, des huiles essentielles ou d'autres extraits de plante.
